# EUROPEAN PATENT APPLICATION

(11) **EP 2 933 337 A1**
(43) Date of publication of application: **21.10.2015**
(21) Application number: 15171198.3
(22) Date of filing: 21.05.2010
(51) Int. Cl.: C12P 1/00, C12M 1/00, C12P 7/10

(54) **METHODS FOR PRETREATING BIOMASS**

(30) Priority: 21.05.2009 US 180308 P
(62) Divisional of application: 10778488.6
(71) Applicant: Board Of Trustees Of Michigan State University, East Lansing, Michigan 48824-1046 (US)
(72) Inventor: Venkatesh, Balan, East Lansing, MI 48823 (US); Dale, Bruce E., Mason, MI 48854 (US); Chundawat, Shishir, Piscataway, NJ 08854 (US); Sousa, Leonardo, Lansing, MI 48912 (US)
(74) Representative: LifeTech IP Spies Danner & Partner Patentanwälte PartG mbB

(57) **Abstract**

A system for treating lignocellulosic biomass, comprising (a) a reaction vessel containing the lignocellulosic biomass and (b) a supply being in connection with the reaction vessel for the delivery of gaseous ammonia or of a mixture of gaseous ammonia and a carrier at an elevated temperature to the reaction vessel, wherein the system is configured to maintain the content of the reaction vessel under pressure, to allow for no expansive release of pressure, and to allow the gaseous ammonia to react with water present in the biomass to produce a treated biomass.

## Description

### CROSS REFERENCE TO RELATED APPLICATIONS

This application claims priority to U.S. Provisional Application No. 61/180,308, filed on May 21, 2009, the contents of which are incorporated by reference in its entirety.

### FIELD OF THE INVENTION

This invention is in the field of biomass processing, in particular alkaline pretreatment of biomass.

### BACKGROUND OF THE INVENTION

With an ever increasing demand for petroleum, there has been growing interest in renewable feedstocks for manufacturing bioethanol (1). Based on recent economic analysis, a modern biorefinery will utilize about 2000 tons/day of lignocellulosic biomass ("biomass") for producing biofuels and biochemicals (2). Lignocellulosic fibers comprise a complex network of cellulose, hemicellulose and lignin (3-4) producing a compact matrix, that is difficult to hydrolyze due to poor enzyme accessibility. To improve accessibility of enzymes to the interwoven polysaccharides, a thermochemical treatment (i.e., a "pretreatment") is typically necessary before enzymatic hydrolysis.

There are different kinds of feed stocks which are readily available for making biofuels. They include agricultural residues, woody biomass, municipal waste, oilseeds/cakes and sea weeds. Commercially available oil seed cakes include canola, sunflower, sesame, peanut, palm oil, Jatropha and soybean. At present these different agricultural residues and oil cakes are either used as animal feed, biocompost materials or are land filled. Grasses and oilseed cakes/meals are rich in protein, fiber and other nutrients. It might be possible to utilize the fiber rich portion of the feed stock in to make bioethanol utilizing a suitable thermochemical pretreatment, enzymatic hydrolysis and fermentation process. Economical pretreatment of feed stocks in a continuous manner is quite challenging. For several leading pretreatment processes like dilute acid, concentrated ammonia (AFEX), steam explosion, and organosolv a detailed economic analysis has been reported (5). Ammonia fiber expansion (AFEX) is a leading alkaline pretreatment process that modifies the cell wall ultra-structure without physically extracting lignin and hemicellulose into a separate liquid stream. In addition, the inhibitory compounds formed during the ammonia pretreatment process are insignificant compared to dilute acid pretreatment which play an important inhibitory role during downstream biological processing. The primary advantage of using ammonia during pretreatment is relatively easy recovery and reusability of ammonia due to its high volatility. Close inspection of various ammonia based pretreatments, reveal that ammonia was either used in its liquid state (30-99% ammonia concentration) (6-11), supercritical state (12) or as dilute ammonium hydroxide (0.1-28%) (13-14). Ammonia recycled percolation (ARP) (15) and AFEX pretreatment are leading ammonia based biomass pretreatment technologies. However, most current pretreatment processes rely on pretreating the biomass using a largely liquid pretreatment medium (with varying ammonia concentrations, 0.1-99%).

Examples of previous ammonia pretreatment processes are ARP and dilute ammonium hydroxide. These processes include: high pretreatment temperature (150-180°C), long residence time (30-120 min), high pressure liquid recycle, separation of biomass into solid and liquid fraction (by separating hemicellulose and lignin from cellulose into liquid fraction), low solids loading, and neutralization and/or recovery needed for downstream processing. Traditionally used gaseous ammoniation includes long residence time (several hours to weeks), and is expensive and inconvenient to scale-up.

In AFEX, anhydrous liquid ammonia is used to pretreat the biomass at relatively low temperatures (70-180°C), intermediate residence times (15-45 min), low moisture (10-200% on dwb), and higher ammonia loading (1:1-3:1, wt of ammonia/wt of biomass). During conventional AFEX, due to gravity, the liquid ammonia flows to the bottom of the reactor. Some amount of the liquid reacts with water and forms ammonium hydroxide and the remaining is converted to gaseous ammonia (depending on the thermodynamic gas-liquid state within the reactor). Since biomass is a poor conductor of heat, it takes a longer residence time (typically 15-45 min) to achieve the desired temperature throughout the reactor. Mixing and uniform pretreatment during AFEX is a significant problem in the absence of a suitable impeller. Mixing solid slurries using propellers and helical impellers is energy intensive and not very effective in reducing mass and heat transfer limitations. In other words, only the biomass which is in contact with ammonium hydroxide and is suitably preheated (i.e. typically biomass close to the walls or at the bottom of the reactor) is pretreated better compared to the bulk of the biomass in the reactor. Another major economic hurdle to the AFEX process is the expensive recovery step, where ammonia needs to be recovered after pretreatment as a gas, recompressed, separated from water and reused as anhydrous liquid ammonia. Also, it is difficult to conduct AFEX in a continuous manner using pressurized liquid ammonia as the pretreatment chemical. The expansive release of ammonia at the end of AFEX pretreatment is energy intensive, generating gaseous ammonia-water mixtures that could make it commercially prohibitive. Supercritical ammonia based pretreatments are much more energy intensive than AFEX making them an economically less viable option.

### BRIEF SUMMARY OF THE INVENTION

The present invention includes a method for treating biomass, comprising the following steps: providing biomass in a reaction vessel; providing gaseous ammonia; delivering the gaseous ammonia to the reaction vessel; allowing time for the gaseous ammonia to react with the biomass in the reaction vessel; and removing the biomass from the reaction vessel.

In certain embodiments of the present invention, the temperature in the reaction vessel may be from about 50 degrees Celsius to about 200 degrees Celsius; or the temperature in the reaction vessel may be from about 50 degrees Celsius to about 100 degrees Celsius; or the temperature in the reaction vessel may increase after delivery of the gaseous ammonia to the reaction vessel.

In other embodiments of the present invention, the gaseous ammonia may be delivered to the reaction vessel at a pressure from about 100 psi to about 1000 psi, from about 200 psi to about 650 psi, or from about 100 psi to about 200 psi.

Further aspects of the inventive method include the gaseous ammonia condensing on the biomass. Additionally, the biomass may include less than about 15% water on a dry weight basis; from about 15% water to about 233% water on a dry weight basis; or the gaseous ammonia reacts with water in the biomass.

In another aspect of the present method, the time for the gaseous ammonia to react with the biomass may be from about 2 hours to about 36 hours, from about 2 hours to about 12 hours, from about 1 minute to about 120 minutes, from about 1 minute to about 20 minutes.

Also, with the present inventive method, the biomass may be uniformly pretreated by the gaseous ammonia; the method may be continuous or semi-batch; and the reaction vessel may be a fixed bed reactor, a fluidized bed reactor, or a semi-fluidized bed reactor.

In some embodiments of the present method, a carrier may be delivered to the reaction vessel, and the carrier may be added to the reaction vessel after the gaseous ammonia gas is delivered to the reaction vessel. The carrier may be combined with the gaseous ammonia, it may be an inert gas, it may be oxidative (e.g., air), and it may be steam. Further, an inert gas and steam may be combined with the gaseous ammonia before the gaseous ammonia is delivered to the reaction vessel.

In a further embodiment, the present method further may include recycling at least a portion of the gaseous ammonia as a gas to be used in the treatment process.

The present invention also includes a method for treating biomass, including: impregnating biomass with ammonia; delivering the biomass to a reaction vessel; providing a gaseous carrier; delivering the gaseous carrier to the reaction vessel; allowing time for the gaseous carrier to react with the biomass in the reaction vessel; and removing the biomass from the reaction vessel.

### BRIEF DESCRIPTION OF THE DRAWINGS

The foregoing summary, as well as the following detailed description of the invention, will be better understood when read in conjunction with the appended drawings. For the purpose of illustrating the invention, there are shown in the drawings and tables, certain embodiment(s) which are presently preferred. It should be understood, however, that the invention is not limited to the precise arrangements and instrumentalities shown.

Figure 1 shows a comparison of conventional AFEX process (I) and gaseous ammonia pretreatment (II). In Figure 1, liquid ammonia is added to the reaction vessel for conventional AFEX treatment (I); and for GAP, the ammonia delivery vessel is heated to transform liquid ammonia to its gaseous state (at pressure PI) and the gaseous ammonia is added to the biomass in the reaction vessel (such that the final pressure in the reaction vessel is P2).

Figure 2 shows enzymatic hydrolysis based glucose yield from corn stover pretreated using AFEX (control) and GAP process at two different residence times as a function of ammonia loading.

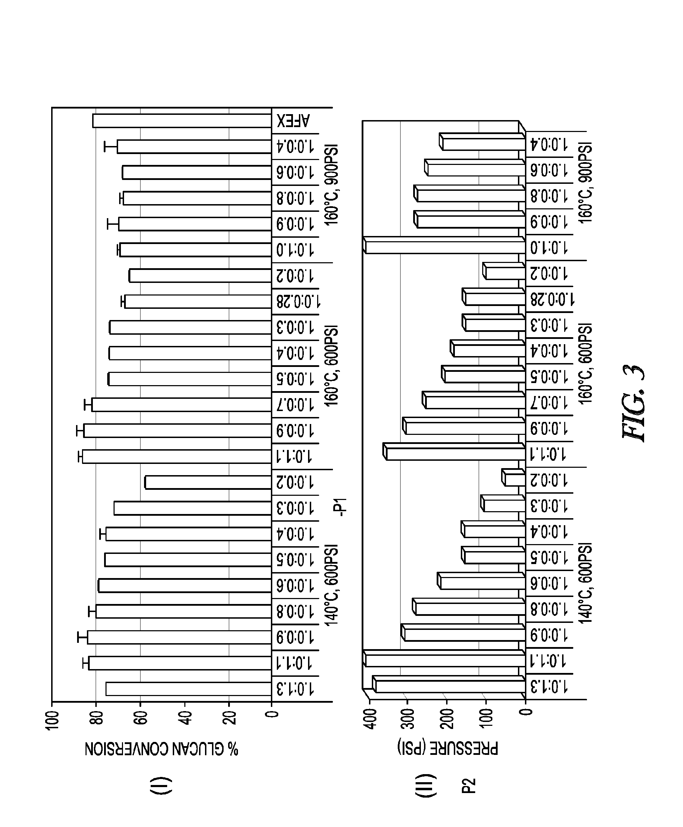

Figure 3 shows percent glucose yield (% glucan conversion) from treated corn stover as a function of different GAP conditions, the effect of ammonia to biomass loading during the GAP process and the pretreatment effect seen during enzymatic hydrolysis (I) and pressure in the reactor during the process (II). In (I), biomass to ammonia loading is shown on x-axis, which is examined at different pressures PI and temperatures (of the gaseous ammonia before adding it to the reaction vessel containing corn stover). Also, in (I), the y-axis gives the over glucose yield achieved as a function of various GAP conditions.

Figure 4 shows the role of explosive removal of ammonia during AFEX and GAP pretreatment process on glucose yield for treated corn stover.

Figure 5 shows the potential application of fluidization during GAP process using gaseous ammonia with or without suitable hot carrier gases.

Figure 6 shows glucose yield for untreated, high moisture (60%, dwb) and low moisture (5%, dwb) AFEX treated corn stover.

Figures 7A-7D show transmission electron micrograph images of untreated and ammonia pretreated corn stover cell walls; Figure 7A, untreated; Figure 7B, low-moisture AFEX treated; Figures 7C and 7D are different portion and magnification of low moisture AFEX treated samples. Untreated corn stover has a distinctinctive multi-lamellar cell wall compared to the nano-porous AFEX treated cell wall. There is a hint of surface deposits on the outer cell wall layers seen as a wavy, amorphous appearance after AFEX.

Figure 8 shows ammonia recovery system and process flow diagram using conventional AFEX (Fig. 8A) and GAP (Fig. 8B) processes.

### DETAILED DESCRIPTION OF THE PREFERRED EMBODIMENTS

Before the subject invention is described further, it is to be understood that the invention is not limited to the particular embodiments of the invention described below. It is also to be understood that the terminology employed is for the purpose of describing particular embodiments, and is not intended to be limiting.

All references, patents, publications, articles, databases, and other writings referred to in this application are incorporated herein by reference in their entirety, as if each were specifically and individually incorporated herein by reference. Such patents, publications, articles, databases, and other writings are incorporated for the purpose of describing and disclosing the subject components of the invention that are described in those patents, publications, articles, databases, and other writings which components might be used in connection with the presently described invention. The information provided below is not admitted to be prior art to the present invention, but is provided solely to assist the understanding of the reader.

The details of one or more embodiments of the invention are set forth in the description below. The preferred embodiments of the present invention may be understood more readily by reference to the following detailed description of the specific embodiments and the Examples included hereafter.

Unless defined otherwise, all technical and scientific terms used herein have the meaning commonly understood by one of ordinary skill in the art to which this invention belongs. Although any methods, devices and materials similar or equivalent to those described herein can be used in the practice or testing of the invention, the preferred methods, devices and materials are now described.

In this specification and the appended claims, the singular forms "a," "an" and "the" include plural reference unless the context clearly dictates otherwise.

The term "ammonia" as used herein means a compound of nitrogen and hydrogen with the formula NH₃.

The term "biomass" as used herein means an organic material, such as wood, plants, and organic wastes, that can be turned into fuel.

The term "gaseous" as used herein means the state of matter distinguished from the solid and liquid states by density, viscosity and/or expansion.

The inventors have developed a process identified as "Gaseous Ammonia Pretreatment" (GAP) in which hot ammonia gas (gaseous ammonia) is used to pretreat biomass in a reaction vessel, such as a reactor, or other vessel that is capable of containing the biomass under pressure. For example, with the GAP process, the contents of the reactor may be maintained at pressures ranging from about 0 psi to about 1000 psi, from about 200 psi to about 500 psi, or from about 100 psi to about 200 psi. In one embodiment, water is used to pre-wet the biomass, the hot ammonia gas is delivered to the biomass under pressure. For example, the gaseous ammonia is delivered to the reaction vessel at pressures ranging from about 0 psi to about 1000 psi, from about 200 psi to about 500 psi, or from about 100 psi to about 200 psi. Then, the hot ammonia gas condenses on the biomass and reacts with water. With this method, the desired temperature (from about 50-200 Degrees Celsius) is achieved instantaneously due to exothermic reaction between water and ammonia. The formation of ammonium hydroxide takes place rapidly where ever water is associated with the biomass. During this process, the biomass is uniformly pretreated by the ammonia (i.e., the majority of the biomass receives about the same pretreatment) and requires short pretreatment time (e.g. from about 1 to about 120 min, or from about 1 to about 20 minutes). This short pretreatment time also helps reduce formation of potentially inhibitory degradation products that might negatively influence downstream biological processing. In some embodiments of the present invention, longer pretreatment times can be used, e.g., from about 2 to about 36 hours, or from about 2 to about 12 hours. Further, with this method, there is no expansive release of pressure at the end of the pretreatment, allowing significant energy savings during recycling of the ammonia.

The process of the present invention can be easily adapted to a continuous method using a stream of (a) recycled ammonia gas, (b) a mixture of recycled ammonia gas and steam, (c) recycled ammonia gas combined with an inert or other carrier gas, or (d) a recycled ammonia/steam gas mixture combined with an inert/carrier gas in any of a fluidized bed reactor, a semi-fluidized, bed reactor, or a fixed bed reactor. It is expected that only a small portion of ammonia (about 0.5 to about 3%, w/w of ammonia/biomass) will be reacted during the present process (due to reaction of ammonia with various cell wall components) and the remaining ammonia (i.e., from about 50% to about 99.5%, from about 75% to about 99.5%, or from about 97% to about 99.5%, w/w of ammonia/biomass) can be recycled in its gaseous state.

In one embodiment, hot ammonia gas is used to treat pre-wetted biomass (from about 15% to about 233% moisture, e.g. water content, dry weight basis) in a reactor with continuous recycling of ammonia- water gaseous mixture (e.g., gaseous ammonia/steam). In another embodiment, a hot ammonia-water gas mixture is used to pretreat either pre- wetted or dry biomass (less than about 15% moisture, dry weight basis, "dwb"), which ammonia- water gas mixture is continuously fed to the reactor and ammonia-water gas mixture is recycled back to the reactor. In a further embodiment, hot ammonia gas is fed to a reactor in combination with a hot inert/carrier gas (e.g. nitrogen, air) to pretreat pre- wetted (from about 15% to about 233% moisture, dry weight basis) or dry biomass (about 15% moisture or less, dwb), which hot ammonia gas is continuously fed to the reactor with recycle of the ammonia- water-inert gas mixture. Alternatively, an oxidative gas, such as air or oxygen, can be combined with the ammonia gas. In another embodiment, a hot ammonia-water gas mixture is fed to a reactor in combination with a hot inert/carrier gas (e.g. steam, nitrogen, air) to pretreat pre-wetted (from about 15% moisture to about 233% moisture, dwb) or dry biomass (about 15% moisture or less, dwb), which hot ammonia-steam gas mixture is continuously fed to the reactor with recycling of the ammonia-steam-inert gas mixture. As provided by this invention, the recycling step is expected to reduce the amount of ammonia necessary to pretreat the biomass. As only a small amount of the gaseous ammonia reacts with the biomass (from about 0.5 to about 3%, w/w of ammonia/biomass), it is expected that a hot inert carrier gas would provide a suitable heat and mass transfer medium replacing expensive ammonia used in current methods (AFEX).

Further, in another embodiment, the carrier gas [either oxidative (e.g., oxygen or air) or non-oxidative (e.g., nitrogen or steam)] are used either combined with gaseous ammonia during the pretreatment process or after the pretreatment process (to remove residual ammonia from the biomass).

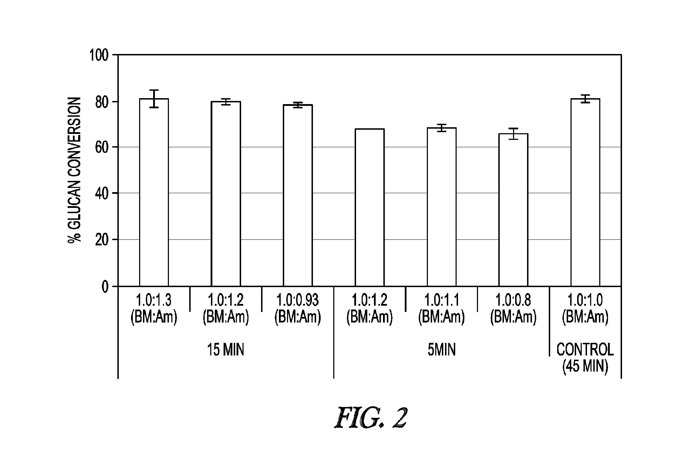

With the present inventive method, effective biomass to ammonia loading is from about 1:0.01 to about 1:5, from about 1:0.2 to about 1:2, or from about 1:0.2 to about 1:1. Further, with the present inventive method, glucan conversion rates are the same, or higher (by about 10-15%) than conversion rates for conventional AFEX. For example, as shown in Figure 2, a 15 minute reaction time with GAP achieves a relatively equivalent conversion rate as a 45 minute reaction time with AFEX. With a 30 minute reaction time with GAP, however, it is expected that the glucan conversion would be increased 10-15% as compared to the AFEX process. Generally, such conversion rates are dependent on other factors, such as, the particular cellulases and hemicellulases, the type and combination of enzymes, and the amount of enzymes used in the enzymatic hydrolysis.

The present invention also includes impregnating biomass with liquid or gaseous ammonia and water (using concentrated/dilute ammonium hydroxide) to achieve lower ammonia loadings (from about 0.01 to about 0.3 kg ammonia per kg biomass) and then feeding the biomass to the reactor continuously where it is pretreated using a hot inert carrier gas (containing little or no ammonia). In one embodiment, this process is performed in a fixed biomass bed reactor with hot ammonia/steam/inert/carrier gas mixtures being purged through the reactor. The gas stream is continuously recycled using compressors and heaters to re-circulate through the fixed biomass bed reactor.

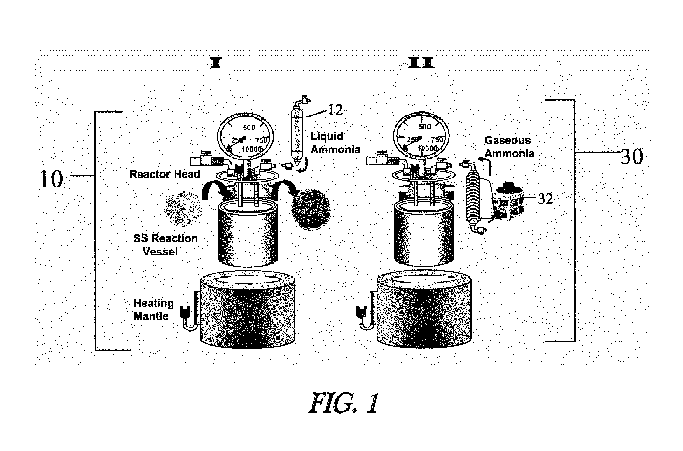

Figure 1 shows a schematic sketch of a comparison of (I) a conventional AFEX apparatus 10 and an apparatus 30 for performing the inventive GAP process (II). Unlike AFEX, where liquid ammonia (delivery vessel pressure at 100-200 psi) is fed to a reactor through the bottom valve of an ammonia delivery vessel 12; in GAP, ammonia in the delivery vessel 32 is pre-heated (to delivery vessel) and fed from the top valve of the delivery vessel 32. This permits the hot ammonia gas to condense on the biomass in GAP (unlike conventional AFEX) thereby causing a fast (e.g., instantaneous) rise in temperature in the reactor. With the AFEX process, it typically takes 15-45 minutes to reach the desired pretreatment temperature in the reactor (e.g. 100 degrees Celsius), after which the temperature is maintained for another 5-45 min. With the AFEX process, typical time pretreating biomass ranges between 20-90 min. In contrast, with GAP (depending on the temperature and pressure PI of the hot ammonia gas fed to the reactor), one can rapidly reach the desired pretreatment temperature in the reactor (e.g. from about 50 to about 200 degrees Celsius, or from about 50 to about 100 degrees Celsius), with a total residence time between about 1 minute to about 120 minutes.

The inventors also have conceived of novel reactor configurations to continuously feed a column based reactor with hot ammonia and/or inert carrier gas mixtures that are recycled and re-fed to the reactor in its gaseous state (without compression of gaseous ammonia to liquid ammonia or ammonium hydroxide mixtures).

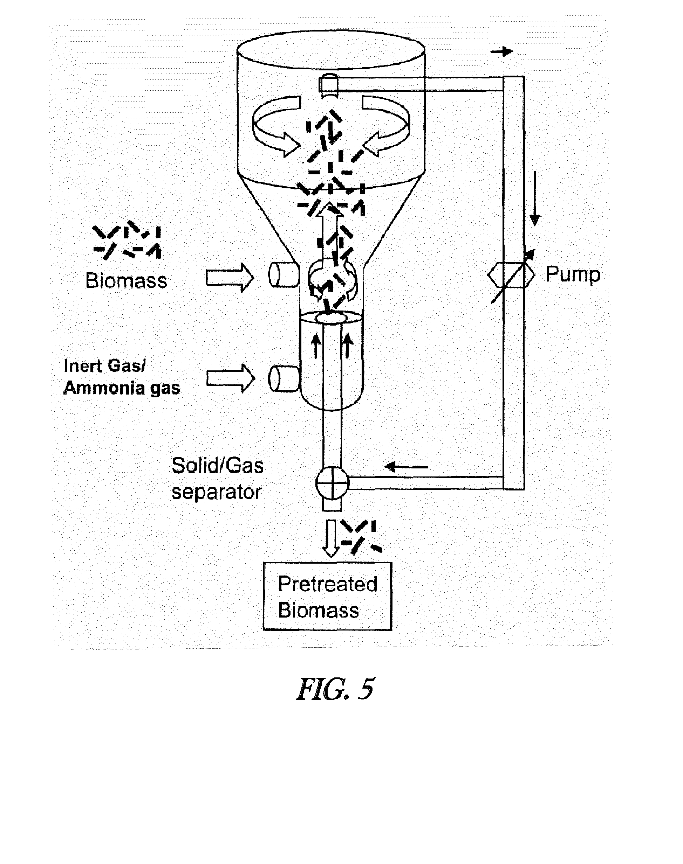

There are several reactor variations that may be used to conduct the GAP process. For example, a semi-batch or continuous reactor with fluidized or semi-fluidized biomass fed continuously into a reactor where the biomass is contacted with hot ammonia and/or inert-carrier gas. In one embodiment, the hot gas may be recovered and recycled. In another embodiment, a batch reactor with a fixed bed of biomass is continuously purged with hot ammonia and/or inert-carrier gas; and the hot gas may be recovered and recycled back into the reactor (see, Figure 5). Figure 5 illustrates one potential process flow schematic for how GAP may be carried out by fluidizing the biomass using gaseous ammonia and other carrier gases. Some of the advantages of fluidized-based treatment are the uniform pretreatment conditions and ease in scaling up as a continuous process along with ease in recycling and reusing hot gaseous ammonia.

With the present GAP biomass pretreatment process, the pretreatment is as homogeneous as possible; and there are negligible mass transfer issues, negligible heat transfer issues, low residence times, low ammonia/water usage, and complex ammonia-water separation procedures are avoided.

Further, as shown in Table 1, the GAP process using a fluidization method will have several advantages as compared to AFEX.

**Table 1:**

| **AFEX** | **Fluidized GAP Process** |
|---|---|
| Liquid bulk phase reaction | Gas bulk phase reaction |
| Mixing with Impellers (non-uniform mixing) | Mixing done by Fluidizing gas (uniform mixing) |
| Use of water (40-100%) (difficult to separate ammonia after pretreatment) | Minimize use of water (<10%)? |
| Preheated liquid ammonia (expensive to recover & liquefy) | Preheated gaseous ammonia (recycle with no liquefaction) |
| Poor mixing (more ammonia-water needed) | Effective mixing (more efficient usage of ammonia-water) |
| Higher re sidence time (15-45 min) | Lower residence time (1-15 min) |
| Hot spots due to non-uniform heating | Homogeneous heating and better control over reaction kinetics |

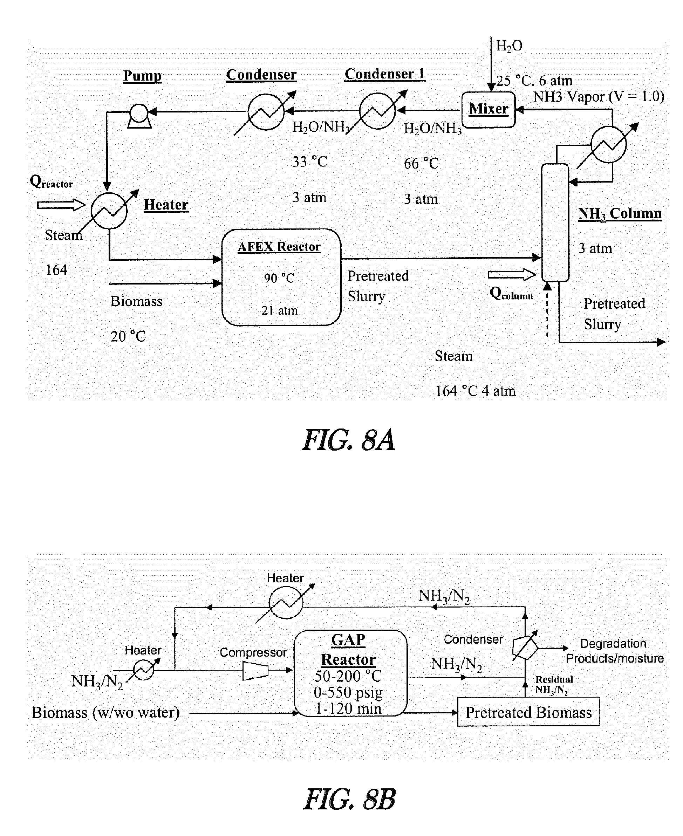

Figures 8A and 8B show a biomass and ammonia/inert gas flow diagram of the recovery process for each of the AFEX and GAP processes, respectively. With respect to the GAP process shown in Figure 8B, the process comprises a GAP Reactor where biomass is fed, with or without water, followed by injecting into the GAP reactor hot ammonia gas(NH₃)/nitrogen(N₂) using a heater and compressor. Most of the hot ammonia gas and nitrogen are recovered after the GAP process and preheated for the subsequent use in the pretreatment process. The residual ammonia/nitrogen present in the pretreated biomass is recovered using a condenser and used for subsequent pretreatment process. The biomass volatiles (degradation products) and moisture along with the residual ammonia is separated from the ammonia recycle stream.

As described herein, there are several pretreatment conditions (temperature of the gaseous ammonia before treatment, pressure PI of the ammonia before delivery, pressure P2 of the ammonia after delivery, reaction time in the GAP reactor, water content of the biomass, and ammonia loading) that can impact the GAP process See, various ranges shown in Figure 8B.

Moreover, the pretreatment conditions are interrelated, i.e., altering one condition may affect another condition. For example, reaction time is dependent on temperature and ammonia pressure. The higher the pressure and temperature of the gaseous ammonia that is delivered to the GAP reactor, the lower the reaction time in the reactor (and the pressure P2 in the reactor would be high as well). Conversely, the lower the pressure PI and temperature of the gaseous ammonia delivered to the reactor, the longer the reaction time in the reactor (and the pressure would be lower in the reactor as well). Also, the diffusion rate of ammonia through the biomass particle is larger with increase in pressure, which means that the reactant can access the reactive bonds much quicker and reduce the total reaction time. In theory, if gaseous ammonia pressure is doubled, the reaction time should decrease by nearly half, since most reactions in the biomass are pseudo-first order. Set- point temperature can also be achieved much quicker with an increase in pressure, since hot gaseous ammonia also has the task to carry heat through the bulk phase to the interior of the biomass, where reactions are happening. For reaction temperatures close to room temperature (i.e., 25-40 degrees Centigrade) reaction times can be extended up to 24 hours (depending on ammonia loading) for achieving close to 90% conversion, while at 100 degrees Centigrade, the total residence time can decrease down to 15 minutes (depending on ammonia loading). In addition to gaseous ammonia pressure and temperature, particle size of the biomass can also affect the reaction time. The smaller the particle size, the faster to achieve set-point temperature and pressure in the interior of the particle, which means that complete conversion should be achieved faster. Finally, the pressure P2 in the reactor can be lowered by decreasing the ammonia fed in to the reactor or by increasing the moisture content of the biomass.

Further, based on varying pretreatment conditions, the inventors found that adding water along with ammonia during the pretreatment process results in two competing reactions; namely, hydrolysis (involving the hydroxyl ion) and ammonolysis (involving the ammonia). The degradation products formed due to hydroxyl ions are mostly acids and are found to be potent inhibitors to microbes in downstream fermentation processes. On the other hand, the ammoniation reaction results in the formation of amides which are found to be significantly less inhibitory to the microbes (unpublished data from Ming W. Lau and Bruce E. Dale). In a typical AFEX process, about 0.5-2 kg water per kg of biomass is used. Because ammonia is soluble in water, it is expensive to distill out ammonia from water after the pretreatment in order to be reused in a continuous biorefinery process.

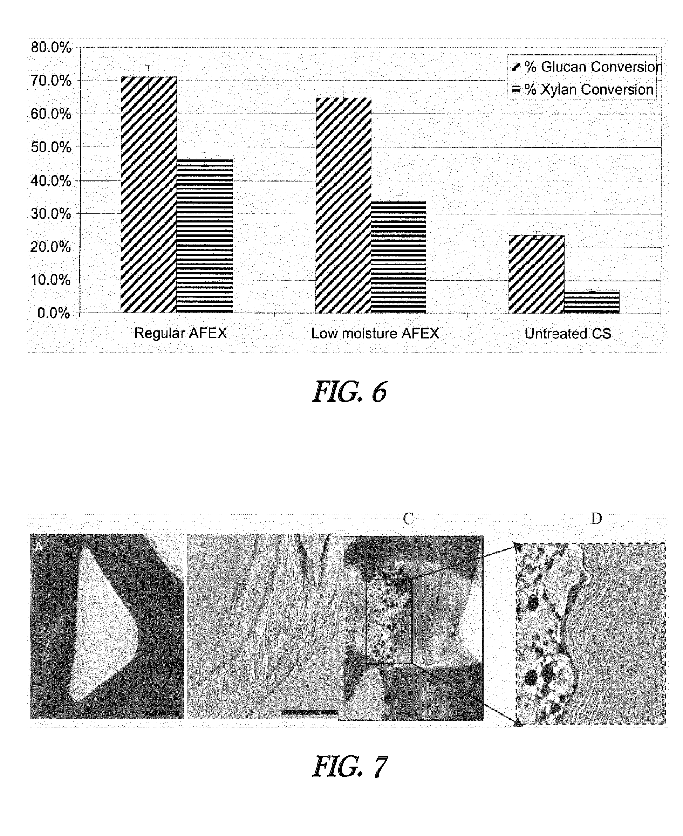

Using the GAP process, the inventors expect that biomass containing from about 5 to about 15% moisture, dwb (the expected moisture content of field dried biomass without external water supplementation during pretreatment) can be pretreated with hot ammonia gas and the percent glucan conversion is similar to that obtained from high moisture (15% or more, dwb) ammonia pretreatment as shown in Figure 6.

The GAP process could be used in a lignocellulosic biorefinery. Specifically, a modern biorefinery will utilize about 2000 tons/day of lignocellulosic biomass for producing biofuels and biochemicals. At present pretreatment, processing costs and green house gas (GHG) emissions are considered as few of the bottle necks for such a biorefinery. Using the GAP process, both pretreatment cost and GHG emissions could be reduced and the technology will be more feasible for the biorefinery. See, e.g., Table 2, hereinbelow.

The GAP process could be used in the edible oilseed and oilcake industry. Oilseeds are typically extracted in two stages: (i) mechanical expeller/press extraction for reducing oil content to 20-25% (w/w), followed by (ii) hexane extraction to remove residual oil (16). The extracted oilcake is then toasted (or desolventized) by steam stripping/cooking to remove residual solvent and pre-conditioned (i.e. to detoxify anti-nutritional components in the oilseed) for animal consumption and/or protein extraction. The pre-conditioning process is generally dependent on the type of oilseed, but typically requires cooking the biomass (at suitable moisture content) with steam at 90-110°C for a period of 15-30 min. The GAP process could be used along with a typical steam toasting process in order to pretreat the biomass prior to subsequent biological processing for producing biofuels and chemicals (e.g. ethanol and biodiesel). The fiber portion of the oilcake could be fermented to ethanol and reacted with the oil extracted from the oilseed to produce biodiesel as well.

One of the major advantages of the AFEX and GAP processes is that, unlike other thermochemical treatments (e.g. dilute acid, organosolv), the temperature severity of pretreatment is fairly low (e.g., 50-150°C for GAP vs. 150-220°C for acidic treatments). Lower temperatures help reduce protein degradation and improve digestibility of important amino acids, like lysine. Ammoniation based treatments are currently employed in the detoxification of oilseeds like groundnuts to remove toxic aflatoxins (17). The inventors have conducted pretreatment of extracted oilseed cakes using a typical AFEX process and hydrolyzed with cellulase enzymes. The AFEX process was found to significantly enhance the rate and yield of maximum achievable sugars compared to the untreated sample (data not shown). The GAP or AFEX pretreatment processes could be used to pretreat oilseed cakes for biomass conversion applications. See, e.g., Balan, V., et al, 2009. Journal of the American Oil Chemists' Society, 86, 157-165.

The GAP process could be used for protein extraction as animal feed. In the Pro- Xan process (18) proteins are extracted from alfalfa through hammer milling to disrupt cell walls followed by juice extraction from screw press and steam injection to coagulate proteins. Finally, solubles are added to press cake and sold as animal feed. In this process, ammonia is used to kill different microbes and to raise the pH (which helps extract protein). Here again, GAP could be used at slightly elevated temperature (instead of room temperature) from about 30°C to about 50-100°C. This will further improve the protein extraction and at the same time pretreat the biomass which could be used in a biorefinery to make biofuels and biochemicals.

The inventors have performed both in vivo and in vitro digestions studies of AFEX treated biomass and found them to be highly digestible. Based on the digestions studies, animals need much less expensive feeds to achieve adequate growth and milk production if these feeds are pretreated by ammonia.

Having now generally described the invention, the same will be more readily understood through reference to the following Examples, which are provided by way of illustration, and are not intended to be limiting of the present invention, unless specified.

### EXAMPLES

### Example 1: Pretreatment oflignocellulosic biomass using gaseous ammonia.

Anhydrous gaseous ammonia was transferred to a stainless steel cylinder and preheated to reach 450-900 psi. In parallel, the biomass with appropriate moisture (60%) was kept in a preheated (at 140 degrees and 160 degrees Centigrade) stainless steel reaction vessel, a vacuum was applied to remove air and to create negative pressure to facilitate ammonia delivery. The preheated ammonia gas was transferred to the reaction vessel. The un-reacted ammonia in the vessel was measured and the actual ammonia added to the pretreatment reactor during the process was calculated. There was a rapid rise in temperature of the biomass (from 30°C initial temperature to about 100-200°C) depending on the pressure/temperature of preheated ammonia gas. The reaction was continued to achieve different residence times and the pressure was then slowly released.

### Example 2: Enzymatic hydrolysis of corn stover pretreated using AFEX (control) and GAP process with different ammonia loading and residence times.

The pretreated biomass was dried in the hood overnight and pretreatment efficiency was determined by digestion of the biomass with commercial enzymes (15 FPU of Spezyme CP from Genencor and 64 pNPGU of beta-glucosidase from Novozyme, per gm glucan) at 50°C over a period of 72 hrs. The hydrolysates were analyzed for glucose using YSI glucose analyzer. Figure 2 shows 5 and 15 minute reaction times using the GAP process, a 45 minute reaction time using the AFEX process, and various ratios of biomass to ammonia. The data in Figure 2 demonstrates equal or better pretreatment efficiency with GAP using significantly shorter reaction times than AFEX.

### Example 3: Biomass glucan conversion as a function of different GAP conditions.

In order to further understand the effect of concentration of ammonia needed during GAP process, the biomass moisture was fixed at 60% and the concentration of biomass to ammonia was varied from 1:1.2 to 1:0.2 (biomass to ammonia loading, w/w). In addition, the ammonia delivery pressure PI (prior to loading) and reactor temperature were varied. These results are shown in Figure 3. From the Figure 3, it is clear that up to 1:0.8 the conversions are comparable to conventional AFEX process (60% moisture, 1:1 biomass to ammonia loading, 45 min. total residence time). By further dropping the biomass to ammonia loading (to 1:0.2) there is only a 10-15% drop in glucose yield compared to the control. That is, there is nearly as much percent glucose conversion for GAP treated corn stover as AFEX treated corn stover at significantly lower ammonia loading and pressure in the reaction vessel. In (II), the y-axis in depicts the pressure in the reactor as a function of GAP conditions and shows that the pressure P2 in the reactor decreases with ammonia loading. By reducing the ammonia to biomass loading, the pressure in the reactor vessel also drops (see Figure 3(II)) to between 50-150 psi.

Though the glucose yield drops by 10%, the pressure P2 in the reactor vessel also drops below 100 psi. Hence, operational and capital costs for GAP carried out at lower pressure (and low ammonia loadings) will be substantially lower compared to AFEX and other ammonia based pretreatments. With the GAP process, by proper selection of an enzyme cocktail (containing suitable cellulases and hemicellulases), the inventors expect that they can further boost the conversion and reduce processing costs by further lowering biomass to ammonia loading (1:0.05-1:0.2 biomass to ammonia loading, dwb) during the GAP process.

### Example 4: Effect of pressure release during pretreatment process.

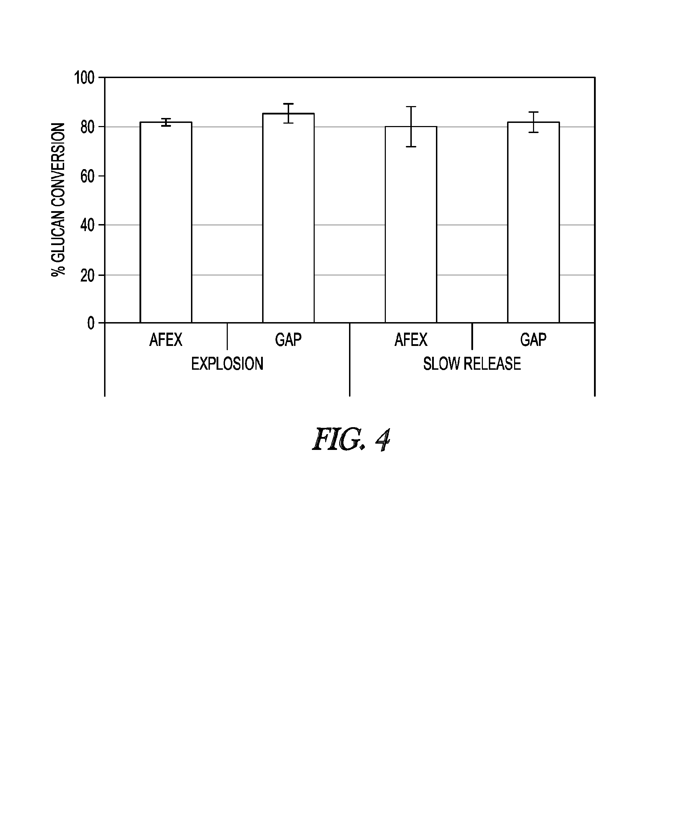

Two independent pretreatments were done using the AFEX and GAP process, utilizing 1:1 biomass to ammonia loading. In the first set of experiments the pressure was released explosively and in the second set of experiments, the pressure was released slowly after the process. In explosive release, the pressure was suddenly reduced (under 1 second) from reaction pressure (200-400 psi) to atmospheric pressure (15 psi). In slow release, the pressure was dropped gradually to atmospheric pressure (over 2 minutes to drop pressure). The resultant feed stock was collected in a tray and dried in hood overnight. The next day treated material was tested for digestibility using commercial enzymes at 50°C, for 72 hrs, as described above (see Figure 4). In Figure 4, the y-axis depicts the % glucose yield (% glucan conversion) for differentially treated biomass samples. The inventors observed marginal decreases in conversion for the pretreatment process performed with slow release as compared to explosion, and this decrease was within the error margin. This indicates that explosive or sudden expansive release of ammonia during pretreatment is unnecessary or not very important. It is therefore possible to continuously pretreat the biomass fed continuously to a constant pressurized reactor fed with hot ammonia gas (and water) and/or inert/carrier gas mixtures.

### Example 5: Hydrolysis for untreated and AFEX-treated corn stover.

Figure 6 shows hydrolysis for untreated and AFEX-treated corn stover. Regular AFEX was performed at 90°C, 1:1 biomass to ammonia loading, at 60% moisture dwb at 5 minutes residence time; and low moisture AFEX was performed at 90°C, 1:1 biomass to ammonia loading at 5% moisture dwb at 5 minutes residence time after 24 hours of incubation at 50°C at 200 rpm.

In order to prove that low moisture biomass (5% moisture) gives comparable pretreatment results to that of high moisture biomass (60% moisture on dwb), the inventors performed pretreatment for these conditions and enzymatic hydrolysis using 15 FPU of cellulase and 64 pNPGU of beta-glucosidase. The conversion results are shown in Figure 6. The y-axis depicts the glucose and xylose yields after enzymatic hydrolysis for the various pretreatment conditions.

In addition, electron tomographic images have shown that pretreating biomass with low moisture creates more porosity within the cell wall than when using higher moisture content (Figure 7A and 7B). The increased porosity would allow better accessibility for the enzymes to hydrolyze pretreated biomass more efficiently. The slightly lower conversion for low moisture AFEX treated sample could be due to lack of suitable hemicellulases during enzymatic hydrolysis and poor heat/mass transfer during AFEX pretreatment.

By proper control of the above-mentioned factors but, instead, using GAP-based fluidization, the inventors expect to obtain better results when compared to regular AFEX conditions. The advantage of low moisture ammonia based treatments, especially during GAP, is the easier recovery of ammonia from water. That is, if there is more the water in the system, it is more expensive it is to recover (and recycle) the ammonia from the system.

### Example 6: Comparison of resource savings and GHG emissions

In order to evaluate the energy, resources saving and green house gas emissions (GHG) for the GAP process when compared to the AFEX process, the inventors performed a calculation based on an Aspen plus model and the results are presented in Table 2. The results show substantial amount of heat, electricity and water saving, in addition to a 3 -fold reduction in GHG emissions for the GAP process.

**Table 2:**

| | **Process information** | | | **CHG** | | |
|---|---|---|---|---|---|---|
| | unit | GAP | AFEX | unit | GAP | AFEX |
| Corn stover | mt | 1 | 1 | | | |
| Ammonia | kg | 8.8 | 8.8 | kg | 24 | 23 |
| Water | kg | 0.0 | 896 | kg | 0 | 1 |
| Electricity | MJ | 19 | 33 | kg | 4 | 7 |
| Heat | MJ | 449 | 2521 | kg | 35 | 194 |
| Blomass* | mt | 1.0 | 1.0 | | | |
| Total | | | | | 63 | 226 |

While the foregoing specification has been described with regard to certain preferred embodiments, and many details have been set forth for the purpose of illustration, it will be apparent to those skilled in the art that the invention may be subject to various modifications and additional embodiments, and that certain of the details described herein can be varied considerably without departing from the spirit and scope of the invention.

### REFERENCES

1. Walter A. (2000), in Industrial uses of biomass energy, edited by Rosillo-Calle F., Bajay SV, Rothman H, pp 200-253, Taylor & Francis.
2. Eggeman T, Elander RT (2005) Process and economic analysis of pretreatment technologies. Bioresour Technol 96:2019-2025.
3. Somerville C, Bauer S, Brininstool G, Facette M, Hamann T, Milne J, Osborne E, Paredez A, Persson S, Raab T, Vorwerk S, Youngs H. (2004) Toward a Systems Approach to Understanding Plant Cell Walls. Science 306:2206-2211.
4. Cosgrove DJ (2005) Growth of the plant cell wall. Nature review 6:850-861.
5. Mosier N, Wyman C, Dale B, Elander R, Lee YY, Holtzapple M, Ladisch M (2005) Features of promising technologies for pretreatment of lignocellulosic biomass. Bioresour Technol 96(6):673-686.
6. Dale BE (1986) Method for increasing the reactivity and digestibility of cellulose with ammonia. US patent No. 4600590.
7. Dale BE (1991) Process for increasing the reacticity of cellulose containing material. US Patent No. 5037663.
8. Dale BE (2000) Process for treating cellulosic materials. US Patent No. 6106888.
9. Dale BE and Weaver JK (2001) Apparatus for treating cellulosic materials. Patent No. US 6176176 B1.
10. Teymouri F, Laureano-Perez L, Alizadeh H, Dale BE (2005) Optimization of the ammonia fiber explosion (AFEX) treatment parameters for enzymatic hydrolysis of corn stover. Bioresource Technol 96:2014-2018.
11. Chundawat PS, Venkatesh B, Dale BE (2007) Effect of Particle Size Based Separation of Milled Corn Stover on AFEX pretreatment and Enzymatic Digestibility. Biotechnol Bioeng 96:219-231.
12. Chou, Y-CT (1987) Supercritical ammonia treatment of lignocellulosic materials. US patent no. 4,644,060.
13. Hennessey SM, Friend J, Dunson JB, Tucker MP, Elander RT and Hames B. (2007) Integration of alternative feedstreams for biomass treatment and utilization. Patent No. US 2007/0037259 A1.
14. Dunson JR, Tucker MP, Elander RT and Lyons RC (2007) System and Process for Biomass treatment. Patent No. US2007/0029252 A1.
15. Kim TH, Lee YY, Sunwoo C, Kim JS. (2006) Pretreatment of corn stover by low- liquid ammonia recycle percolation process. Appl. Biochem. Biotechnol. 133:41-57.
16. Erickson DR (1990) Edible fats and oil processing-Basic principle and modern practices. AOCS Press, Netherlands.
17. Pivai G, Galvanoz F, Pietril A, Piva A (1995) Detoxification Methods of Flatoxins-A Review. Nutrition Research, 15(5):767-776.
18. Prevot-D'Alvise N, Lesueur-Lambert C, Fertin-Bazus A, Fertin B and Dhulster P (2003) Development of a pilot process for the production of alfalfa peptide isolate. J. Chem. Technol. Biotechnol. 78:518-528.

## Claims

1. A system for treating lignocellulosic biomass, comprising:
(a) a reaction vessel containing the lignocellulosic biomass; and
(b) a supply being in connection with the reaction vessel for the delivery of gaseous ammonia or of a mixture of gaseous ammonia and a carrier at an elevated temperature to the reaction vessel;
wherein the system is configured to:
(i) maintain the content of the reaction vessel under pressure;
(ii) allow for no expansive release of pressure; and
(iii) allow the gaseous ammonia to react with water present in the biomass to produce a treated biomass.

2. The system of claim 1, wherein the reaction vessel is selected from the group consisting of a fixed bed reactor, a fluidized bed reactor, and a semi-fluidized bed reactor.

3. The system of claim 2, wherein the reactor is a semi-batch or a continuous reactor.

4. The system of claim 1, wherein the gaseous ammonia is delivered to the reaction vessel at a pressure between 100 psi and 1000 psi.

5. The system of claim 1, wherein the content of the reaction vessel is maintained at a pressure between 100 psi and 1000 psi.

6. The system of claim 1, wherein the system is configured to allow for no expansive release of pressure at the end of the treatment.

7. The system of claim 1, wherein the system is configured to allow for a slow release of pressure over two minutes.

8. The system of claim 7, wherein the pressure is released from 200-400 psi to 15 psi.

9. The system of claim 1, wherein the gaseous ammonia or the mixture of gaseous ammonia and carrier is delivered by an injection supply.

10. The system of claim 9, wherein the injection supply for the delivery of gaseous ammonia or of a mixture of gaseous ammonia and a carrier comprises a heater and compressor.

11. The system of claim 1, wherein the carrier is selected from the group consisting of an oxidative carrier, an inert gas or steam.

12. The system of claim 1, further comprising: a condenser for the recovery of residual ammonia or residual ammonia/carrier from the biomass.

13. Use of a system of claim 1 in a lignocellulosic biorefinery for the production of biofuels and biochemicals.

14. Use of a system of claim 1 for the preconditioning of oilseeds.
